# EUROPEAN PATENT APPLICATION

(11) **EP 3 456 313 A1**
(43) Date of publication of application: **20.03.2019**
(21) Application number: 16901174.9
(22) Date of filing: 29.12.2016
(51) Int. Cl.: A61K 8/9789, A61K 8/92, A61Q 19/08

(54) **COSMETIC COMPOSITIONS FOR SKIN REPARATION**

(30) Priority: 12.05.2016 US 201662335397 P
(71) Applicant: Natura Cosméticos S.A., 05106-000 São Paulo SP (BR)
(72) Inventor: ROSA FERRARI, Cintia, 05106-000 São Paulo - SP (BR); IATESTA DOMENICO, Carolina, 05106-000 São Paulo - SP (BR); BELTRAME REIGADA, Juliana, 05106-000 São Paulo - SP (BR); PEDROSO DE OLIVEIRA, Ana Paula, 05106-000 São Paulo - SP (BR); CASTELLANI, Debora Cristina, 05106-000 São Paulo - SP (BR); BRÁS COSTA, Camila, 05106-000 São Paulo - SP (BR); ZIMBARDI, Daniela, 05106-000 São Paulo - SP (BR); SOLDATI, Pedro Paulo, 13209-460 Jundiaí - SP (BR); MALANCONI THOMAZ, Fernanda, 04559-010 São Paulo - SP (BR)
(74) Representative: Abel & Imray
(86) International application number: PCT/BR2016/050362
(87) International publication number: WO 2017/193186

(57) **Abstract**

This invention refers to cosmetic compositions for skin repair comprising at least one oil selected from *Bertholletia excelsa* or *Fevillea trilobata* and cosmetically acceptable carriers.

This invention also refers to the use of the said compositions in cosmetic treatment for skin regeneration and/or healing, anti-aging treatment, treatment of skin sensitized by esthetic procedures and/or dry skin and the modulation of cellular processes involved in skin repair, associated with skin regeneration and/or healing mechanisms.

## Description

### FIELD OF THE INVENTION

The present invention relates to skin repair cosmetic compositions comprising at least one oil selected from *Bertholletia excelsa* or *Fevillea trilobata* and cosmetically acceptable carriers, as well as the use thereof and skin regeneration and healing methods, that serve as anti-aging treatment, treatment of skin sensitized by esthetic procedures and dry skin, through modulating cellular processes involved in skin regeneration and healing mechanisms. It also encompasses an *in vitro* method for assessing skin repair.

### PRIOR ART

The skin has an incredible ability to regenerate constantly. On average, each 28 days a cell travel from the junction of the dermis and the epidermis up to the *stratum corneum,* and is then shed as a dead cell.

This regeneration capacity is adversely affected to a substantial extent by several environmental and physiological factors, such as moisture loss, the actions of external agents such as sun, pollutants, air humidity, diseases, smoking and direct damage to the surface of the *corneus* layer (such as burns, scrapes, cracks, etc.).

Adverse effects on skin regeneration capacity may also be due to aging, as cell regeneration slows down together with cell life cycle speed.

At around 20 years of age, the skin starts to produce less moisture, losing its elasticity as the first fine lines begin to appear, particularly those caused by UV rays.

At around 30 years of age, skin regeneration capacity begins to decrease, as conjunctive tissue alterations reduce skin elasticity and resistance, together with its ability to retain water. During this phase, fine lines and crepey skin become more marked, and wrinkles begin to develop.

At 40 years of age, skin cell division and blood flow drops significantly, and the skin becomes drier, with wrinkles appearing around the eyes and lips, as well as on the forehead.

From 50 years of age onwards, the skin becomes even more sensitive, fragile and less flexible, due particularly to hormonal changes. Wrinkles and pigmentation spots become deeper and more intense.

This is why skin repair products have become increasingly more important at the various skin phases.

Skin repair takes place in two ways: through regeneration and healing. These processes are classified as inflammatory by nature. When an inflammatory process begins, this is always a defense mechanism against an aggressive agent, also triggering mechanisms that underpin healing and regeneration.

Regeneration may be defined as the process through which cells killed by aggression are replaced by parenchyma cells from the same organ. Healing occurs through a complex multi-step process that is highly organized and dynamic, involving several cell types that are important for restoring and maintaining tissue integrity.

Skin repair processes (regeneration or healing) may occur even when there are no lesions visible to the naked eye, such as microlesions that occur after exposure to the environment (UV, weather dryness, aging). This includes the initial signaling steps (that may be ascertained through FGF-2 measurement), inflammation (which may be ascertained through measuring inflammatory cytokines (IL-1B, IL-6, IL-8, IL-10, IL-12 p70), cell proliferation and migration (which may be ascertained through cell proliferation assays), and remodeling and resolution (which may be ascertained through measuring MMP-13 or the enzymatic activities of collagenase and elastase).

Although some products - such as madecassoside, allantoin, papain or lucuma nut oil - have been proposed, there is still a need for cosmetic compositions with proven skin repair effects, particularly for topical administration.

### DESCRIPTION OF THE INVENTION

In an initial aspect, this invention refers to cosmetic compositions for skin repair comprising at least one oil selected from *Bertholletia excelsa* (known as Brazilnut) or *Fevillea trilobata* (known as crabwood (*nhandiroba*) and cosmetically acceptable carriers.

The cosmetic compositions for skin repair according to the present invention may be presented in different cosmetic forms, including but not limited to, emulsions, gels, powders and sticks, among others, including cosmetically appropriate carriers known at the state of the art for the selected cosmetic form.

These compositions provide anti-aging treatment (anti-aging), including wrinkle reduction and encouraging the regenerative capacity of the skin, enhancing firmness and elasticity, particularly when reduced by age, as well as treatment of skin sensitized by esthetic procedures, such as depilation, laser, peeling or dermo-abrasion and skin-drying treatment (oiliness control).

The cosmetic compositions for skin repair according to the present invention are suitable for use on the face and body.

The cosmetic compositions according to the present invention are proven to be efficient for modulating the cellular processes involved in skin repair, particularly in skin regeneration and healing mechanisms, including FGF-2 that indicates the start of the healing process, IL-1B, IL-6, IL-8, IL-10 and IL-12 inflammatory cytokines, cell proliferation and migration associated with barrier construction and elastase activity (MMP-1 2) that degrades elastin, MMP-13, glycosaminoglycans and elastin.

Consequently, another aspect of this invention also addresses the use of the cosmetic compositions for skin repair according to the present invention and methods related to cosmetic treatment for skin regeneration and healing, particularly through modulating the cellular processes involved in skin repair, associated with skin regeneration and healing mechanisms, including FGF, IL-1B, IL-6, IL-8, IL-10, IL-12, cell proliferation and migration, and the activities of elastase, MMP-13, glycosaminoglycans and elastin.

In another aspect, this invention also refers to an *in vitro* method of assessing skin repair, that is suitable for studying a variety of active ingredients, comprised of the following steps:
- Step 1 - Initial Indication: quantification of the FGF-2 growth factor through the flow cytometry technique;
- Step 2 - Inflammation: quantification of inflammatory cytokines (IL-1B, IL-6, IL-8, IL-10, IL-12 p70) through the flow cytometry technique;
- Step 3 - Cell proliferation and migration:
   - Cell proliferation through the Neutral Red detection method;
   - Cell migration through the ECIS and Scratch method;
- Step 4 - Remodeling and Resolution
   - Quantification of matrix metalloproteinase MMP-13 through the Enzyme-Linked Immunosorbent Assay (ELISA) technique;
   - Determination of collagenase/gelatinase enzyme activity (MMP-1 and MMP-3) and elastase (MMP-1 2) through the enzyme inhibition assay.

The following examples illustrate this invention, without imposing any constraints thereon.

### EXAMPLES

All the tests were conducted in triplicate, with experiments repeated at least twice, using negative control groups (no stimulation with active ingredients) and positive control groups (stimulated with molecules modulating the marker to be assayed).

Furthermore, all the tests were conducted at a baseline condition stimulated by only the active ingredient being tested (*Bertholletia excelsa* oil and *Fevillea trilobata* oil) or the standard (madecassoside, allantoin, papain or lucuma nut oil) and a condition stimulated with molecules that are known to be released during each one of the steps in the healing process (such as NGF and IL-1-beta) together with stimulation by the active ingredient being tested (*Bertholletia excelsa* oil and *Fevillea trilobata* oil) or the standard (madecassoside, allantoin, papain or lucuma nut oil).

The tissue regeneration process (healing) may be divided into four sequential steps the final objective of which is the restoration of cell architecture and function. Thus, some key events were defined in each of these steps in order to characterize this process and its respective measurement *in vitro.*

As a result, this project encompassed the following *in vitro* assays:
- Step 1 - Initial Indication: quantification of the FGF2 growth factor through the flow cytometry technique;
- Step 2 - Inflammation: quantification of inflammatory cytokines (IL-1B, IL-6, IL-8, IL-10, IL-12 p70) through the flow cytometry technique;
- Step 3 - Cell proliferation and migration:
   - Cell proliferation through the Neutral Red detection method;
   - Cell migration through the ECIS and Scratch method;
- Step 4 - Remodeling and Resolution
   - Quantification of matrix metalloproteinase MMP-13 through the Enzyme-Linked Immunosorbent Assay (ELISA) technique;
   - Determination of collagenase/gelatinase enzyme activity (MMP-1 and MMP-3) and elastase (MMP-1 2) through the enzyme inhibition assay.

### QUANTIFICATION OF FGF-2 (Fibroblast Growth Factor)

Comprising a family of 22 polypeptides, FGFs regulate the proliferation, differentiation, migration and survival of different cell types. Their functions are performed through activating four transmembrane receptor tyrosine kinases called FGFR1-4.

Earlier studies disclosed important roles played by FGFs in skin development, homeostasis and repair. Some of them are expressed in this tissue, and many are overexpressed after an injury. Consequently, in order to boost fibroblast proliferation stimulation and angiogenesis, their potential has been widely studied for healing modulation use. Furthermore, among all the FGFs, skin healing and regeneration studies have been conducted primarily with FGF2, also known as basic FGF (bFGF). This molecule is a powerful mitogen for mesodermal and neuroectodermal origin, including fibroblasts and endothelial cells.

In this assay, a significant (p<0.0001) increase of up to 60 times was noted for treatment with *Bertholletia excelsa* oil and up to 152 times for treatment with *Fevillea trilobata* oil, in comparison to the negative control. These increases were similar to those observed for standard madecassoside (increase of up to 115 times) and lucuma nut oil (increase of up to 134 times).

### QUANTIFICATION OF INFLAMMATORY CYTOKINES

Cytokines are small secreted proteins that affect the behavior of immune cells, and other cells as well. They include interleukins, lymphokines and some related signaling molecules such as Tumor Necrosis Factor-Alpha (TNF-alpha) and interferons. Chemokines (or chemotactic cytokines) comprise a subgroup of cytokines that stimulate chemotaxis and leukocyte spillovers to injury sites.

Pro-inflammatory cytokines, including IL-1 alpha and IL-1 beta, IL-6 and TNF-alpha, play an important role in repairing lesions through inducing biological responses at lesion sites, including the stimulation of keratinocyte and fibroblast proliferation, MEC synthesis and extracellular matrix protein degradation, fibroblast chemotaxis and immune response regulation. However, it must be stressed that the production of these cytokines must take place at low levels, and must be combined with the production of anti-inflammatory cytokines such as IL-10, the main cytokine for resolving inflammatory processes at lesion sites. Corroborating this information, all the increases noted for the pro-inflammatory cytokines in this study were at low but significant levels, and while combined with a marked increase in the IL-10 anti-inflammatory cytokine.

Corroborating this context, in brief, it was possible to detect significant modulation in the pro- IL-1 beta, IL-6 and IL-8 inflammatory cytokines and the IL-10 and IL-12 anti-inflammatory cytokines. For treatments with *Fevillea trilobata* oil, an increase was observed of 2 times (p<0.001), 2.5 times (p<0.01) and 2.7 times (p<0.0001), respectively, in terms of negative control; for treatments with lucuma nut oil an increase was observed of 3.5 times (p<0.0001), 3 times (p<0.01) and 4.2 times (p<0.0001) respectively, in terms of negative control. For standard madecassoside, a twofold increase (p<0.05) was detected only in the IL-8 levels, in terms of negative control. All these treatments also caused combined increases in IL-10 anti-inflammatory cytokine levels: *Fevillea trilobata* oil and *Bertholletia excelsa* oil (increase of up to 3.3 times in terms of negative control, p<0.0001); madecassoside (increase of 2.3 times in terms of negative control, p<0.0001) and lucuma nut oil (increase of 3.3 times in terms of negative control, p<0.0001).

### CELL PROLIFERATION

In this test, significant increases were shown in cell proliferation of up to twofold for treatments with *Bertholletia excelsa* oil (p<0.001) and between 80% and 100% for treatments with *Fevillea trilobata* oil (p<0.001), compared to the untreated control after 48 hours of treatment. Standard madecassoside, allantoin, papain and lucuma nut oil did not present significant increases in cell proliferation.

### CELL MIGRATION

In this test, it was possible to calculate cell migration rates and migration times for each of the evaluated treatments. For the cell migration rate, a significant increase of 38% was noted for treatment with *Bertholletia excelsa* oil compared to the negative control (p<0.001). However, treatment with *Fevillea trilobata* oil indicated a slight drop in the migration rate (p<0.05) compared to the negative control. The standards do not present significant differences for this parameter.

In terms of migration times after treatments, only *Bertholletia excelsa* oil presented a potential drop (p<0.01) in the function under assessment.

### ACTIVITIES OF COLLAGENASE (MMP-1 COLLAGEN I)/GELATINASE (MMP-2 COLLAGEN IV), ELASTASE (MMP-12) AND QUANTIFICATION OF MMP-13 (COLLAGENASE III)

The proteins constituting the extracellular matrix consist of collagen, adhesive glycoproteins (fibulin, fibrillin, elastin), proteoglycans and others. Through structural domains, these proteins promote biological activities, modulate cytokine and growth factor activity, and constitute recognition sites for interacting with specific cell membrane receptors. These proteins form structural scaffolding in all tissues, integrating with each other in the construction of large structural networks. The synthesis, deposition and remodeling of these proteins are essential for restoring damaged tissues during the healing process.

Collagen is the most abundant protein in the human body, with the literature containing descriptions of at least 19 different types of collagen. In the skin, collagen is predominantly type I and III in a 4:1 proportion, ensuring skin structure, strength and integrity. Type IV collagen is a component of endothelial and epidermal basal membranes.

During the initial healing phases, collagen III predominates, and is replaced by collagen I in the final phases. During the final phase, MMP-11 and MMP-13 are released by endothelial cells and fibroblasts, which allows the cells to migrate, while neutrophil and macrophage proteases remove degraded matrix components in order to remodel the repaired tissue.

The activity of collagenase (MMP-1)/gelatinase (MMP-2) was not altered in any of the evaluated treatments; however, elastase activity (MMP-12) dropped during treatments with *Fevillea trilobata* oil (p<0.0001) and *Bertholletia excelsa* oil (p<0.05), as well as standard lucuma nut oil (p<0.0001), demonstrating the potential of these active ingredients for reducing protein degradation in the elastin elastic system.

Furthermore, threefold increases in MMP-13 quantification were noted in the treatments with *Bertholletia excelsa* oil (p<0.01), compared to the untreated control, as well as the standard madecassoside, allantoin, papain and lucuma nut oil (p<0.01).

After conducting these studies, it was possible to identify through this set of tests that *Bertholletia excelsa* and/or *Fevillea trilobata* oils are endowed with biological activity related to skin repair/regeneration (Table 1), in a manner similar to that noted for some standards such as madecassoside. In comparative terms, *Bertholletia excelsa* oil also presented greater potential for modulating this activity than *Fevillea trilobata* oil. Consequently, these oils may be associated with products recommended for skin sensitized by the esthetic procedures, damaged skin (com microlesions), and dry skin or as an anti-aging agent, as the regenerated capacity of the skin drops gradually with advancing age.

In addition to the novel benefit identified for *Bertholletia excelsa* and *Fevillea trilobata* oils, an *in vitro* methodology was used to assess this benefit that is suitable for other active ingredients of interest.

Furthermore, the combination of results obtained for some of the tests in this study indicates that *Bertholletia excelsa* and *Fevillea trilobata* oils are also endowed with other anti-aging benefits for the skin (Table 2). In comparative terms, *Fevillea trilobata* oil presented greater potential for this benefit than *Bertholletia excelsa* oil.

**Table 1. Combination of tests for the skin regeneration/repair benefit and indication of concentrations presenting significant results**

| Test | *Bertholletia excelsa* oil | *Fevillea trilobata* oil |
|---|---|---|
| FGF-2 Dosage | 0.2 mg/mL | 0.1 mg/mL and 0.2 mg/mL |
| Cytokines Dosage Pro/Anti-inflammatory Balance | 0.1 mg/mL and 0.2 mg/mL | 0.1 mg/mL and 0.2 mg/mL |
| IL-12 Reduction | 0.2 mg/mL | - |
| Cell Proliferation | 0.1 mg/mL | 0.1 mg/mL |
| Cell Migration | 0.1 mg/mL | 0.1 mg/mL and 0.2 mg/mL |
| | | |
| Collagenase / Gelatinase Activity | - | - |
| Elastase Activity | 0.2 mg/mL | 0.2 mg/mL |
| MMP-13 Dosage | 0.2 mg/mL | - |

| | | |
|---|---|---|
| Key: - (results not significant) | | |

**Table 2. Combination of tests for the skin anti-aging benefit and indication of concentrations presenting significant results**

| Test | *Bertholletia excelsa* oil | *Fevillea trilobata* oil |
|---|---|---|
| Cytokines Dosage (anti-inflammatory) | 0.1 mg/mL and 0.2 mg/mL | 0.1 mg/mL and 0.2 mg/mL |
| Elastase Activity | 0.2 mg/mL | 0.2 mg/mL |
| Elastin Dosage | NA | 0.01 mg/mL, 0.2 mg/mL and 0.4 mg/mL |
| GAGs Dosage | NA | 0.2 mg/mL |

| | | |
|---|---|---|
| Key: NA (not assessed) | | |

These results show that the compositions addressed by this invention are proven to be efficient (*in vitro*) for modulating cellular processes involved in skin repair, particularly in skin regeneration and healing mechanisms, including FGF-2, IL-1B, IL-6, IL-8, IL-10 and IL-12 inflammatory cytokines, cell proliferation and migration and elastase activity (MMP-1 2), MMP-13, glycosaminoglycans and elastin, resulting in an anti-aging effect, including wrinkle reduction and promoting the regenerative capacity of the skin, fostering firmness and elasticity as well as treating skin sensitized by esthetic procedures, and dry or damaged skin.

A person skilled in the art would promptly assess the advantages of the invention through the teachings in the text and the examples presented, proposing equivalent variations and alternative embodiments without extending beyond the scope of the invention, as defined in the Claims appended hereto.

## Claims

**1.** COSMETIC COMPOSITIONS FOR SKIN REPAIR **characterized in that** it comprises at least one oil selected from *Bertholletia excelsa* or *Fevillea trilobata* and cosmetically acceptable carriers.

**2.** Compositions, according to Claim 1, **characterized in that** they are used in skin regeneration and/or healing treatment.

**3.** Compositions, according to Claim 2, **characterized in that** they are used in anti-aging treatment, treatment of skin sensitized by esthetic procedures and/or dry skin.

**4.** Compositions, according to Claim 1, **characterized in that** they act on the modulation of the cellular processes involved in skin repair, associated with skin regeneration and/or healing mechanisms, including FGF-2, IL-1B, IL-6, IL-8, IL-10, IL-12, cell proliferation and migration, and the activities of elastase, MMP-13, glycosaminoglycans and/or elastin.

**5.** USE OF THE COMPOSITIONS, according to one of Claims 1 to 4, **characterized in that** this is for cosmetic treatment in skin regeneration and/or healing.

**6.** USE OF THE COMPOSITIONS, according to one of Claims 1 to 4, **characterized in that** this is for anti-aging treatment, treatment of skin sensitized by esthetic procedures and/or dry skin.

**7.** USE OF THE COMPOSITIONS, according to one of Claims 1 to 4, **characterized in that** this is for the modulation of cellular processes involved in skin repair, associated with skin regeneration and/or healing mechanisms, including FGF-2, IL-1B, IL-6, IL-8, IL-10, IL-12, cell proliferation and migration, and the activities of elastase, MMP-13, glycosaminoglycans and/or elastin.

**8.** COSMETIC TREATMENT METHOD FOR SKIN REGENERATION AND HEALING **characterized in that** it consists of applying an effective amount of the cosmetic compositions defined in one of Claims 1 to 4 to the face or body skin.

**7.** MODULATION METHOD FOR CELLULAR PROCESSES INVOLVED IN SKIN REPAIR, associated with skin regeneration and/or healing mechanisms FGF-2, IL-1B, IL-6, IL-8, IL-10, IL-12, cell proliferation and migration, and the activities of elastase, MMP-13, glycosaminoglycans and/or elastin **characterized in that** it consists of applying an effective amount of the cosmetic compositions defined in one of Claims 1 to 3 to the face or body skin.

**8.** *IN VITRO* SKIN REPAIR ASSESSMENT METHOD **characterized in that** it comprises the following steps:
• Step 1 - Initial Indication: quantification of the FGF-2 growth factor through the flow cytometry technique;
• Step 2 - Inflammation: quantification of inflammatory cytokines (IL-1B, IL-6, IL-8, IL-10, IL-12 p70) through the flow cytometry technique;
• Step 3 - Cell proliferation and migration:
- Cell proliferation through the Neutral Red detection method;
- Cell migration through the ECIS and Scratch method;
• Step 4 - Remodeling and Resolution
- Quantification of matrix metalloproteinase MMP-13 through the Enzyme-Linked Immunosorbent Assay (ELISA) technique;
- Determination of collagenase/gelatinase enzyme activity (MMP-1 and MMP-3) and elastase (MMP-1 2) through the enzyme inhibition assay.
